# EUROPEAN PATENT SPECIFICATION

(11) **EP 0 642 334 B1**
(45) Date of publication and mention of the grant of the patent: **18.08.1999**
(21) Application number: 93911039.1
(22) Date of filing: 03.05.1993
(51) Int. Cl.: A61K 9/20, A61K 9/00

(54) **PHARMACEUTICAL AND OTHER DOSAGE FORMS**
PHARMAZEUTISCHE UND ANDERE DARREICHUNGSFORMEN
PRESENTATIONS PHARMACEUTIQUES ET AUTRES FORMES GALENIQUES

(30) Priority: 06.05.1992 US 879754
(43) Date of publication of application: 15.03.1995
(73) Proprietor: JANSSEN PHARMACEUTICA INC., Titusville, New Jersey 08560-0200 (US)
(72) Inventor: GOLE, Dilip, J., Ann Arbor, MI 48105 (US); WILKINSON, Paul, K., Ann Arbor, MI 48103 (US); DAVIES, J., Desmond, Grosse Pointe Farms, MI 48236 (US); LEVINSON, R., Saul, Chesterfield, MO 63005 (US)
(74) Representative: Cockbain, Julian, Dr.
(86) International application number: US9304201
(87) International publication number: WO9323017

(56) References cited:
- EP-A- 0 450 141
- WO-A-91/09591

## Description

The present invention relates to methods for preparing products by removal of a solid frozen solvent from a frozen matrix mixture.

Freeze-drying, i.e., lyophilization, is a well known method of drying heat-sensitive materials in order to protect them from thermal damage. In the past, preparations containing active ingredients, such as pharmaceuticals, nutrients, diagnostics, fertilizers and insecticides, have been prepared by freeze-drying aqueous solutions or suspensions containing these bioactive ingredients. Conventional methods of freeze drying or lyophilization involve the freezing of a material at a very low temperature followed by a dehydration by sublimation under high vacuum.

One problem that has arisen, however, with the use of conventional freeze-drying processes is cracking of the freeze-dried preparations. Typically, cracking is caused by the stresses set up during ice crystallization. Though cracking is never desirable, it is especially undesirable where drop methods of freezing are employed. In such cases, cracking of the frozen droplets usually results in unusable and inelegant remnants of fractured droplets.

Another problem encountered by use of known freeze-drying methods is a phenomenon called meltback. Meltback occurs when the heat required during the drying process melts the frozen material. As such, meltback defeats the whole purpose of freeze-drying-the removal of water through sublimation as opposed to evaporation. To avoid meltback in conventional freeze-drying methods, only limited amounts of material of limited thickness can be dried at one time or, alternatively, very low temperatures have to be used, thereby considerably extending the time required for sublimation. Even with these limitations, conventional freeze-drying methods are not always sufficient to prevent meltback.

Yet another problem inherent in conventional freeze-drying methods is a lack of resistance to disintegration in freeze-dried materials, i.e., they have little strength.

Freeze-drying methods generally yield products that merely crumble when handled. Various freeze-drying and packaging methods have been employed in attempts to circumvent this problem. For example, U.S. Patent No. 4,305,502 describes a method for forming a shaped article by a lyophilization process in a depression in a sheet of film material. However, such packaging techniques do not avoid the problems posed by conventional freeze-drying methods; the tablets are still susceptible to crumbling if transferred to other packaging.

In the area of pharmaceuticals, known freeze-dried dosage forms do not always exhibit fast dissolution rates when brought into contact with appropriate solvents, such as water, saliva or gastrointestinal fluids. Rapid dissolution of pharmaceutical dosage forms can be of critical importance in instances where it is desirable that the pharmaceutical enter the physiological system as soon as possible. For example, many individuals, particularly pediatric and geriatric patients, experience difficulty and discomfort in swallowing solid, slow dissolving tablets and capsules. Similar difficulties are encountered when administering pharmaceuticals orally to animals in the veterinary treatment of those animals.

Various methods for freeze-drying pharmaceutical dosage forms by lyophilization have been developed to provide fast dissolving dosage forms. U.S. Patents Nos. 2,166,074; 3,234,091; 4,371,516 and 4,302,502 and United Kingdom Patents Nos. 698,767 and 1,310,824 are all concerned with freeze-dried dosage forms that are able to dissolve rapidly. In addition, U.S. Patent No. 4,642,093 teaches a procedure for preparing a freeze-dried (lyophilized) foam dosage form using conventional lyophilization techniques that results in rapidly dissolving pharmaceutical dosage forms. Yet another problem intrinsic to conventional lyophilization methods is the lack of uniform porosity in the lyophilized product. Uniform porosity in a lyophilized product is critical for post-loading a dosage form with an active agent. Thus, there is a need for a method of producing a dosage form that will avoid cracking and meltback and having adequate strength and porosity and exhibiting a fast speed of dissolution upon ingestion.

Alternative drying methods to lyophilization are disclosed in WO 91/09591 and EP-A-450141. In both cases, the methods disclosed involve removal of water from frozen aqueous solutions by immersion in a water-miscible solvent such as anhydrous ethanol. This procedure is referred to in WO 91/09591 as "solid state dissolution".

### Description of the Invention

Viewed from one aspect the invention provides a solid lyophilised dosage form comprising a porous network of matrix material that disperses rapidly in water, the matrix material comprising at least about 0.1% by weight of a matrix forming agent selected from the group consisting of gelatin, pectin, soy fiber protein and mixtures thereof and one or more amino acids having from about 2 to 12 carbon atoms, said matrix forming agent and said one or more amino acids being present in said dosage form in a weight ratio of from 10:1 to 1:5.

Viewed from a further aspect the invention provides a method of producing a solid lyophilised dosage form comprising a porous network of matrix material that disperses rapidly in water, which method comprises obtaining a matrix material solution containing a matrix forming material, and subjecting said solution to lyophilisation, said matrix material comprising at least about 0.1% by weight of a matrix forming agent selected from the group consisting of gelatin, pectin, soy fiber protein and mixtures thereof and one or more amino acids having from about 2 to 12 carbon atoms, and said matrix forming agent and said one or more amino acids being present in said matrix material solution in a weight ratio of from 10:1 to 1:5.

In a preferred embodiment of the method of the invention of the present invention, the matrix material solution used to form the inventive dosage form contains from about 0.1% to about 15% matrix material by weight. Prererably the matrix material solution comprises from about 0.1% to about 3% of the matrix forming agent by weight, from about 0.5% to about 10% of the one or more amino acids by weight, and from about 0.5% to about 10% mannitol by weight.

Any active or bioactive agent contained in the dosage form may be advantageously present in a coated form. In this embodiment, the active or bioactive agent is present in particulate form and the particles of the agent are coated with an appropriate coating agent(s) to protect the active or bioactive agent from process solvents, the aqueous environment of the oral or other mucosal cavity, or environmental conditions that would dissolve or deteriorate said active or bioactive agent. These coating materials may be selected from natural or synthetic polymers that are either hydrophilic or hydrophobic in nature or other hydrophobic materials such a fatty acid, glycerides, triglycerides and mixtures thereof. In this way, the taste of the active or bioactive agent may be masked, while at the same time permitting the solid dosage form to dissolve rapidly upon contact with physiological solvents. Examples of active agents that may be coated in accordance with the present invention include acetaminophen, ibuprofen, chlorpheniramine maleate, pseudoephedrine and dectromethorphan.

The dosage forms of the present invention are quite robust in comparison to prior art dosage forms. Also, the inventive dosage forms exhibit greatly reduced or no shrinkage under high temperature or humidity conditions when compared to prior art dosage forms, especially those prepared using lyophilisation. The dosage forms of the invention disperse rapidly in water, e.g. in less than 10 seconds.

It is a further object of the present invention to provide a solid carrier system for chemicals that a user may add to a medium to instantaneously obtain a solution or dispersion of desired concentration. It is a further additional object of the present invention to provide dosage forms that include active ingredients, such as pharmaceuticals, nutrients, diagnostics, confectioneries, fertilizers and insecticides.

The dosage forms of the present invention are produced with minimal cracking or meltback of the processed sample. They exhibit rapid dissolution in appropriate solvents, having uniform porosity and adequate strength of handling, i.e. resistance to disintegration or crumbling under normal manufacturing and handling conditions.

The dosage forms of the present invention and in particular those containing glycine as one of the matrix components have the following advantages : quick dissolution and disintegration, pleasant taste and mouthfeel, nutritional value, low calorie content and noncariogenicity. In the realm of pharmaceutical use, the present dosage forms exhibit rapid dissolution upon contact with physiological solvents, such as water, saliva, or gastrointestinal fluids. Therefore, the present inventive pharmaceutical dosage forms provide a more rapid dispersion of the pharmaceutical within the body upon ingestion.

Pharmaceutical applications comprise dosage forms having mucoadhesive properties or designed to deliver a drug at a controlled rate; dosing units designed to deliver drugs in the eye, in vaginal, rectal and other body orifices; solid dosage forms designed to replace liquid formulations; dry medicated preparations for topical application after resolvation (reconstitution); preparation of medicated units or sheets for topical application; preparation of more palatable dosage forms of drugs that exhibit disagreeable organoleptic properties: dosage forms for oral delivery of drugs to persons who have difficulty swallowing tablets or capsules.

Embodiments of the present invention can be used in various applications. Applications in the food industry comprise preparation of and presentation of dried products composed of food materials; to provide a method for the selective extraction of a material in the solid form during the drying process; preparation of confectionery products; preparation of dosing units for the purpose of modifying properties (e.g. taste, colour etc.) or quality of drinking water. Veterinary applications comprise the preparation of dosing units for veterinary use; the preparation of aquarium care and feed products. As cosmetic applications there may be mentioned the preparation of dry systems for medical and cosmetic use after resolvation. Diagnostic applications comprise enzyme/cofactors and biochemical carrier systems. Sanitary applications are, for example, the preparation of dosing units for water purification or the preparation of fragrance carrier units for personal, household and industrial use. Other applications comprise reconstitutable carrier units for pigmented application for paint and other artistic uses; agriculture and horticulture products requiring release of active ingredients in the presence of water or rain; the preparation of easily removable mold or model material or the preparation of easily removable space maintenance and/or alignment aid for construction or manufacturing.

Though the following description focuses on the inclusion of pharmaceuticals as the active agents, it is to be understood that the desirable properties of the inventive methods and dosage forms may be advantageously used in connection with many different types of active agents.

The various ingredients that may be incorporated into the initial mixture may include matrix forming agents and secondary components. Matrix forming agents suitable for use in the present invention include materials derived from animal or vegetable proteins, such as the gelatins, dextrins and soy, wheat and psyllium seed proteins; gums such as acacia, guar, agar, and xanthan; polysaccharides; alginates; carboxymethylcelluloses; carrageenans; dextrans; pectins; synthetic polymers such as polyvinylpyrrolidone; and polypeptide/protein or polysaccharide complexes such as gelatin-acacia complexes.

Other matrix forming agents suitable for use in the present invention include sugars such as mannitol, dextrose, lactose, and galactose; cyclic sugars such as cyclodextrin; inorganic salts such as sodium phosphate, sodium chloride and aluminum silicates; and amino acids having from 2 to 12 carbon atoms such a glycine, L-alanine, L-aspartic acid, L-glutamic acid, L-hydroxyproline, L-isoleucine, L-leucine and L-phenylalanine. Persons having skill in the art will recognize other acceptable matrix forming agents that may be employed in the present invention.

One or more matrix forming agents may be incorporated into the solution or suspension prior to solidification. The matrix forming agent may be present in addition to a surfactant or to the exclusion of a surfactant. In addition to forming the matrix, the matrix forming agent may aid in maintaining the dispersion of any active ingredient within the solution or suspension. This is especially helpful in the case of active agents that are not sufficiently soluble in water and must, therefore, be suspended rather than dissolved.

Secondary components such as preservatives, flavors, antioxidants, surfactants, sweeteners, viscosity enhancers, or colorings may also be incorporated in the formulation. Other secondary components include the active or bioactive agents to be dosed or delivered. These active agents may include pharmaceuticals, nutrients, vitamins, minerals, diagnostics, fertilizers and insecticides. Examples of pharmaceutical agents that may be incorporated in the initial mixture are chlorpheniramine maleate, pseudoephedrine, detromethorphan, meclizine dihydrochloride, haloperidol, albuterol sulfate, dimenhydrinate, and benzodiazepines such as diazepam, lorazepam and congeners thereof. However, virtually any pharmaceutical agent may be used in connection with the present invention, either by adding the pharmaceutical to the mixture to be solidified or by post loading the pharmaceutical onto a preformed placebo delivery matrix or dosage form.

The speed in which the sample prepared by the inventive method dissolves is dependent in large part on the choice of matrix forming agent(s) and their concentration. In particular, dosage forms of the size mentioned in the examples described hereinafter, will dissolve or disperse quite rapidly, for example, in less than about 10 seconds or even faster e.g. in less than about 5 seconds or even less, e.g. about 3 seconds. Compounds (either alone or in combination) that can be used as a matrix forming material for producing placebos or matrixes comprise hydroyethylcellulose, sodium carboxymethylcellulose, microcrystalline cellulose, corn syrup solids, maltrins (maltodextrins), polydextroses, pectins, carrageenan, agar, chitosan, locust bean gum, xanthan gum, tragacanth, guar gum, konjac flour, rice flour, wheat gluten, sodium starch glycolate, gelatin (pharmaceutical or food grade), soy fiber protein, potato protein, papain, horse radish peroxidase, glycine, mannitol, cyclodextrins (including Beta-cyclodextrin and hydroxypropyl beta-cyclodextrin),sucrose, xylitol, galactose, dextrose, polygalacturonic acid, magnesium aluminum silicate, magnesium trisilicate or natural clays.

Preferred matrix forming agents include pharmaceutical grade gelatins, pectins (nonhydrolyzed, partially hydrolyzed or hydrolyzed), glycine and mannitol, either alone or in combination.

In a further aspect of the present invention, there are provided improved solid dosage forms of the type comprising a porous network of matrix material that disperses rapidly in water, in particular in less than about ten seconds. The matrix material in these dosage forms is made up from at least about 0.1% by weight of a matrix forming agent selected from the group consisting of gelatin, pectin, soy fiber protein and mixtures thereof, and one or more amino acids having from about 2 to 12 carbon atoms. The latter amino acids comprise, for example, glycine, L-alanine, L-aspartic acid, L-glutamic acid, L-hydroxyproline, L-isoleucine, L-leucine and L-phenylalanine.

A preferred combination of matrix forming agents is gelatin and one or more amino acids having from 2 to 12 carbon atoms, especially glycine. In these combinations, the amino acid(s) are present relative to the solvent used to form the matrix solution in a ratio of about 1:18 to about 1:180, in particular of about 1:30 to about 1:100, by weight on a wet basis. These matrix materials may further comprise sugars such as mannitol, dextrose, lactose, galactose trehalose and cyclic sugars such as cyclodextrins or substituted cyclodextrins, in particular mannitol. A particularly preferred combination of matrix forming agent is gelatin, glycine and mannitol.

The solution or suspension of which the present dosage forms are made may further contain the secondary components mentioned before and also xanthan gum and polyacrylic acid polymers and salts thereof (also referred to as carbomers or carboxyvinyl polymers, e.g. carbopol™), which may be added e.g. to increase viscosity.

The ratio between the materials in these combinations may vary within certain ranges. In particular the ratio of the quantity of gelatin, pectin or soy fiber protein to amino acid varies from about 10:1 to 1:5, in particular from 5:1 to 1:3 or more in particular from 3:1 to 1:1. A preferred ratio is 1.5:1. The ratio of the amount of mannitol to gelatin, pectin, soy fiber protein or mixtures thereof is in the range of 5:1 to 1:5, in particular from 2:1 to 1:2. A preferred ratio is 1.5/2.

The solution or dispersion of materials for preparing the matrix conveniently contains from 0.1% to 15% by weight of gelatin, pectin, soy fiber protein or mixtures thereof, in particular from 1% to 3% more in particular from 1.2% to 2.5%. It further conveniently contains from 0.1% to 10%, in particular from 1% to 2.5% by weight of amino acid and from 0.1% to 10%, in particular from 1% to 3.0% of mannitol, the remainder being solvent. The percentages mentioned in this paragraph all are by weight. Typically the weight ratio of the solvent or dispersion to the non-solvent components is in the range of about 5:1 to 50:1, in particular from about 10:1 to 30:1, for example about 20:1.

Various concentrations of matrix forming agents may be used in the present invention. Preferred concentrations of matrix forming agents in a suitable solvent are about 0.1 to 15% weight/weight (w/w). A more preferred concentration is about 0.5 to 4% (w/w). Optimum results are obtained from the present inventive method in pharmaceutical applications when an approximately 2% weight/weight aqueous solution of a given matrix forming agent is used.

The concentration of secondary components incorporated in the initial mixture are limited primarily by the solubility of the secondary component(s) in the solvent used to dissolve the component. The concentration required is defined by the amount of agent to be incorporated in the dosage form. Therefore, concentrations of these components in the initial mixtures may range from about 0.0001 to 20%.

Dosage forms may be prepared in a wide variety of sizes through use of the present invention, ranging from about 0.25 ml or g to 30 ml or g and larger.

For lyophilisation, it is advantageous to freeze the matrix material solution in molds that are coated or lined for easy release of the frozen material. Preferred molds are made from talc-filled polypropylene with a layer of silicone/simethicone baked on the surface(s) in contact with the matrix material solution.

The invention is illustrated further by the following examples, which are not to be construed as limiting the invention in scope or spirit to the specific procedures described in them.

### EXAMPLE 1

Gelatin (pharmaceutical grade) (15g), mannitol (20g), aspartame (20g), L-alanine (10 g) were dissolved in 935 g of water with constant stirring. The resulting solution was carefully transferred into 1g size molds. The molds and their contents were cooled with dry ice for about one hour or frozen quickly in a cold gas freezing tunnel. The water in the ice state was removed by lyophilization. This produced a sample, i.e. a network of carrier material, that disintegrated rapidly when taken orally. Each of the processed samples weighed 65 mg. This process can be repeated by replacing the alanine with L-aspartic acid, L-glutamic acid, L-hydroxyproline, L-isoleucine, L-leucine and L-phenylalanine, either alone or in combination.

### EXAMPLE 2

Formula A: Pectin (20g) was dissolved in water with heating and constant stirring. The resulting solution was autoclaved at 121°C for 15 minutes. The autoclaved solution was then allowed to attain room temperature. The autoclaved solution was then dried by a suitable method. The dry autoclaved pectin was then washed with ethanol. The ethanol washed pectin was filtered and dried by suitable method. The washed pectin powder was used in Formula B.
Formula B: The dry ethanol washed pectin (12g), pectin (5g), mannitol (20g), aspartame (5g) and L-alanine (20g) were dissolved in 938g of purified water with constant stirring. The resulting solution was processed as in Example 1. The processed sample weighed 62 mg and dissolved rapidly in water and the mouth.

### EXAMPLE 3

Mannitol (20 g), L-glutamic acid (25g), and aspartame (5g) were dissolved in 942g of purified water. Soy fiber protein (30 g) and xanthan gum (0.5 g) were dispersed in solution. The resulting dispersion was processed as in Example 1. This produced a sample, i.e. a network of carrier material, that disintegrated rapidly in one to five seconds when taken orally. Each of the processed samples weighed 58 mg.

### EXAMPLE 4

Gelatin (pharmaceutical grade) (15g), glycine (10g), mannitol (20g), aspartame (13.3 g), glutamic acid (2 g), D&C yellow #10 (0.02 g), and FD&C red #40 (0.02 g) were dissolved in purified water (564.3 g) with heating and constant stirring. The resulting solution was then allowed to attain room temperature. To 624.6 g of this solution, 3 g of spray-dried orange, 1 g of natural and artificial prosweet, and 11 g of artificial raspberry flavors were added with constant stirring. The mixture was stirred until uniform dispersion was obtained.

The fine acetaminophen powder, consisting of the active ingredient encapsulated in a hydrophobic matrix such as fatty acid(s)/glycerides, was then suspended uniformly in the flavor mixture. The resulting suspension was transferred to 1.5 ml size molds The molds and their contents were frozen in a cold gas freezing tunnel.

The water in the ice state was removed by lyophilization. This produced a sample, i.e., a network of carrier material that disintegrated rapidly when taken orally. Each of the processed units contained 250 mg of acetaminophen.

### EXAMPLE 5

Gelatin (pharmaceutical grade) (15 g), glycine (10 g), mannitol (20 g), aspartame (13.3 g), glutamic acid (2 g), D&C yellow #10 (0.02 g), and D&C green #5 (0.02 g) were dissolved in purified water (597.9 g) with heating and constant stirring. The resulting solution was then allowed to attain room temperature. To 644.9 g of this solution, 7.5 g of honey-lemon-lyptus and 1 g of natural and artificial flavors were added with constant stirring. The mixture was stirred until uniform dispersion was obtained.

The fine ibuprofen powder, consisting of the active ingredient encapsulated in a hydrophobic matrix such as fatty acid(s)/glycerides, was then suspended uniformly in the flavor mixture. The resulting suspension was transferred to 1.5 ml size molds. The molds and their contents were frozen in a cold gas freezing tunnel.

The water in the ice state was removed by lyophilization. This produced a sample. i.e., a network of carrier material that disintegrated rapidly when taken orally. Each of the processed units contained 200 mg of ibuprofen.

### EXAMPLE 6

Gelatin (pharmaceutical grade) (15 g), glycine (10 g), mannitol (20 g), aspartame (13.3 g), glutamic acid (2 g), FD&C green #3 (0.01 g), and FD&C red #40 (0.02 g) were dissolved in purified water (761.7 g) with heating and constant stirring. The resulting solution was then allowed to attain room temperature. To 822 g of this solution, 3 g of artificial grape and 1 g of natural and artificial prosweet flavors was added with constant stirring. The mixture was stirred until uniform dispersion was obtained.

The fine phenylopropanolamine hydrochloride/chlorpheniramine maleate powder, consisting of the active ingredient encapsulated in a hydrophobic matrix such as fatty acid(s)/glycerides, was then suspended uniformly in the flavor mix.

The resulting suspension was transferred to 0.5 ml size molds. The molds and their contents were frozen in a cold gas freezing tunnel.

The water in the ice state was removed by lyophilization. This produced a sample, i.e., a network of carrier material that disintegrated rapidly when taken orally. Each of the processed units contained 25 mg of phenylpropanolamine hydrochloride and 4 mg of chlorpheniramine maleate.

### EXAMPLE 7

Gelatin (pharmaceutical grade) (15 g), glycine (10 g), mannitol (20 g) aspartame (13.3 g) and glutamic acid (2 g), were dissolved in purified water (665.5 g) with heating and constant stirring. The resulting solution was then allowed to attain room temperature. To 725.8 g of this solution, 5 g of methollyptus flavor was added with constant stirring. The mixture was stirred until uniform dispersion was obtained. The fine pseudoephedrine hydrochloride powder, consisting of the active ingredient encapsulated in a hydrophobic matrix such as fatty acid(s)/glycerides, was then suspended uniformly in the flavor mixture. The resulting suspension was transferred to 1.5 ml size molds. The molds and their contents were frozen in a cold gas freezing tunnel.

The water in the ice state was removed by lyophilization. This produced a sample. i.e., a network of carrier material that disintegrated rapidly when taken orally. Each of the processed units contained 60 mg of pseudoephedrine hydrochloride.

## Claims

1. A solid lyophilised dosage form comprising a porous network of matrix material that disperses rapidly in water, the matrix material comprising at least about 0.1% by weight of a matrix forming agent selected from the group consisting of gelatin, pectin, soy fiber protein and mixtures thereof and one or more amino acids having from about 2 to 12 carbon atoms, said matrix forming agent and said one or more amino acids being present in said dosage form in a weight ratio of from 10:1 to 1:5.

2. A dosage form according to claim 1 wherein the amino acids are selected from glycine, L-aspartic acid, L-glutamic acid, L-hydroxyproline, L-isoleucine, L-leucine and L-phenylalanine.

3. A dosage form according to either of claims 1 and 2 additionally containing a sugar selected from mannitol, dextrose, lactose, galactose, trehalose, cyclodextrins, and substituted cyclodextrins.

4. A dosage form according to claim 3 comprising gelatin and wherein the amino acid is glycine and the sugar is mannitol.

5. A dosage form according to any of claims 1 to 4 additionally comprising an active agent.

6. A dosage form according to claim 5 which additionally comprises an active agent in particulate form, particles of the active agent being coated with a coating agent.

7. A dosage form according to claim 6 wherein the coating agent is a fatty acid, a glyceride, triglyceride or a mixture thereof.

8. A dosage form according to any of claims 1 to 7 wherein the dosage form disperses in water in less than about 10 seconds.

9. A dosage form according to any of claims 1 to 8 additionally containing xanthan gum or polyacrylic acid polymers or salts thereof.

10. A method of producing a solid lyophilised dosage form comprising a porous network of matrix material that disperses rapidly in water, which method comprises obtaining a matrix material solution containing a matrix forming material, and subjecting said solution to lyophilisation, said matrix material comprising at least about 0.1% by weight of a matrix forming agent selected from the group consisting of gelatin, pectin, soy fiber protein and mixtures thereof and one or more amino acids having from about 2 to 12 carbon atoms, and said matrix forming agent and said one or more amino acids being present in said matrix material solution in a weight ratio of from 10:1 to 1:5.

11. A method as claimed in claim 10 wherein said matrix material solution comprises about 0.1% to about 15% matrix material relative to the weight of the solution.

12. A method as claimed in claim 11 wherein said matrix material solution comprises about 0.1% to about 3% of said matrix forming agent by weight.

13. A method as claimed in claim 11 wherein said matrix material solution comprises about 0.5% to about 10% of said one or more amino acids by weight and about 0.5% to about 10% mannitol by weight.

14. A method according to any of claims 10 to 13 wherein the dosage form without active ingredient is first prepared and subsequently loaded with a predetermined quantity of the active ingredient.

15. A method according to any of claims 10 to 14 wherein the matrix material solution additionally comprises a gas dispersed therethrough to form a foam dosage form.

16. The dosage form or method according to any preceding claim wherein said weight ratio of matrix forming agent to said one or more amino acids is from 3:1 to 1:1.

17. The dosage form or method according to claim 16 wherein said weight ratio is from 1.5:1 to 1:1.

18. The dosage form or method according to any one of claims 1 to 15 wherein said weight ratio of matrix forming agent to said one or more amino acids is from 1.5:1 to 1:5.

## Patentansprüche

1. Feste lyophilisierte Darreichungsform mit einem porösen Matrixmaterialnetzwerk, das rasch in Wasser dispergiert, wobei das Matrixmaterial mindestens ungefähr 0,1 Gew.-% eines Matrixbildners aus der Gruppe Gelatine, Pectin, Soyafaserprotein und deren Mischungen sowie eine oder mehrere Aminosäuren mit ungefähr 2 bis 12 Kohlenstoffatomen enthält, wobei der Matrixbildner und die eine oder mehreren Aminosäuren in der Darreichungsform in einem Gewichtsverhältnis von 10:1 bis 1:5 vorliegen.

2. Darreichungsform nach Anspruch 1, wobei die Aminosäuren aus der Gruppe Glycin, L-Asparaginsäure, L-Glutaminsäure, L-Hydroxyprolin, L-Isoleucin, L-Leucin und L-Phenylalanin ausgewählt sind.

3. Darreichungsform nach einem der Ansprüche 1 und 2, die weiterhin einen Zucker aus der Gruppe Mannit, Dextrose, Lactose, Galactose, Trehalose, Cyclodextrine und substituierte Cyclodextrine enthält.

4. Darreichungsform nach Anspruch 3, die Gelatine enthält und bei der es sich bei der Aminosäure um Glycin und bei dem Zucker um Mannit handelt.

5. Darreichungsform nach einem der Ansprüche 1 bis 4, die zusätzlich einen Wirkstoff enthält.

6. Darreichungsform nach Anspruch 5, die zusätzlich einen teilchenförmigen Wirkstoff enthält, wobei die Teilchen des Wirkstoffs mit einem Überzugsmittel beschichtet sind.

7. Darreichungsform nach Anspruch 6, wobei es sich bei dem Überzugsmittel um eine Fettsäure, ein Glycerid, Triglycerid oder eine Mischung aus diesen handelt.

8. Darreichungsform nach einem der Ansprüche 1 bis 7, die in weniger als ungefähr 10 Sekunden in Wasser dispergiert.

9. Darreichungsform nach einem der Ansprüche 1 bis 8, die weiterhin Xanthangummi oder Polyacrylsäurepolymere oder deren Salze enthält.

10. Verfahren zur Herstellung einer festen lyophilisierten Darreichungsform mit einem porösen Matrixmaterialnetzwerk, das rasch in Wasser dispergiert, mit den folgenden Schritten: Bildung einer einen Matrixbildner enthaltenden Matrixmateriallösung und Lyophilisieren dieser Lösung, wobei das Matrixmaterial mindestens ungefähr 0,1 Gew.-% eines Matrixbildners aus der Gruppe Gelatine, Pectin, Soyafaserprotein und deren Mischungen sowie eine oder mehrere Aminosäuren mit ungefähr 2 bis 12 Kohlenstoffatomen enthält und wobei der Matrixbildner und die eine oder mehreren Aminosäuren in der Matrixmateriallösung in einem Gewichtsverhältnis von 10:1 bis 1:5 vorliegen.

11. Verfahren nach Anspruch 10, bei dem die Matrixmateriallösung ungefähr 0,1% bis ungefähr 15% Matrixmaterial bezogen auf das Gewicht der Lösung enthält.

12. Verfahren nach Anspruch 11, bei dem die Matrixmateriallösung ungefähr 0,1 Gew.-% bis ungefähr 3 Gew. -% des Matrixbildners enthält.

13. Verfahren nach Anspruch 11, bei dem die Matrixmateriallösung ungefähr 0,5 Gew.-% bis ungefähr 10 Gew. -% der einen oder mehreren Aminosäuren und ungefähr 0,5 Gew.-% bis ungefähr 10 Gew.-% Mannit enthält.

14. Verfahren nach einem der Ansprüche 10 bis 13, bei dem zuerst die Darreichungsform ohne Wirkstoff hergestellt und anschließend mit einer vorbestimmten Wirkstoffmenge beladen wird.

15. Verfahren nach einem der Ansprüche 10 bis 14, bei dem die Matrixmateriallösung weiterhin ein eindispergiertes Gas enthält, wodurch eine schaumförmige Darreichungsform gebildet wird.

16. Darreichungsform oder Verfahren nach einem der vorhergenden Ansprüche, bei der bzw. dem das Gewichtsverhältnis zwischen Matrixbildner und der einen oder mehreren Aminosäuren 3:1 bis 1:1 beträgt.

17. Darreichungsform oder Verfahren nach Anspruch 16, bei der bzw. dem das Gewichtsverhältnis von 1,5:1 bis 1:1 beträgt.

18. Darreichungsform oder Verfahren nach einem der Ansprüche 1 bis 15, bei der bzw. dem das Gewichtsverhältnis zwischen Matrixbildner und der einen oder mehreren Aminosäuren 1,5:1 bis 1:5 beträgt.

## Revendications

1. Forme galénique solide lyophilisée comprenant un réseau poreux de matériau matriciel qui se disperse rapidement dans l'eau, le matériau matriciel comprenant au moins environ 0,1% en poids d'un agent formant la matrice choisi parmi le groupe constitué de la gélatine, de la pectine, de la protéine de fibre de soja et de leurs mélanges, et un ou plusieurs aminoacides ayant d'environ 2 à 12 atomes de carbone, ledit agent formant la matrice et lesdits un ou plusieurs amino-acides étant présents en un rapport pondéral de 10:1 à 1:5.

2. Forme galénique selon la revendication 1, dans laquelle des amino-acides sont choisis parmi la glycine, l'acide L-aspartique, l'acide L-glutamique, la L-hydroxyproline, la L-isoleucine, la L-leucine et la L-phénylalanine.

3. Forme galénique selon l'une ou l'autre des revendications 1 et 2, contenant en outre un glucide choisi parmi le mannitol, le dextrose, le lactose, le galactose, le tréhalose, des cyclodextrines et des cyclodextrines substituées.

4. Forme galénique selon la revendication 3, comprenant de la gélatine et dans laquelle l'aminoacide est la glycine et le glucide est le mannitol.

5. Forme galénique selon l'une quelconque des revendications 1 à 4, comprenant en outre un agent actif.

6. forme galénique selon la revendication 5, qui comprend en outre un agent actif sous forme particulaire, les particules de l'agent actif étant enrobées d'un agent d'enrobage.

7. Forme galénique selon la revendication 6, dans laquelle l'agent d'enrobage est un acide gras, un glycéride, un triglycéride ou un mélange de ceux-ci.

8. Forme galénique selon l'une quelconque des revendications 1 à 7, la forme galénique se dispersant dans l'eau en moins d'environ 10 secondes.

9. Forme galénique selon l'une quelconque des revendications 1 à 8, contenant en outre de la gomme de xanthane ou des polymères de l'acide polyacrylique ou leurs sels.

10. Méthode de production d'une forme galénique solide lyophilisée comprenant un réseau poreux de matériau matriciel qui se disperse rapidement dans l'eau, laquelle méthode comprend les étapes consistant à obtenir une solution de matériau matriciel contenant un matériau formant la matrice et à soumettre ladite solution à une lyophilisation, ledit matériau matriciel comprenant au moins environ 0,1% en poids d'un agent formant la matrice choisi parmi le groupe constitué de la gélatine, de la pectine, de la protéine de fibre de soja et de leurs mélanges et un ou plusieurs aminoacides ayant d'environ 2 à 12 atomes de carbone, ledit agent formant la matrice et lesdits un ou plusieurs amino-acides étant présents dans ladite solution de matériau matriciel en un rapport pondéral de 10:1 à 1:5.

11. Méthode selon la revendication 10, dans laquelle ladite solution de matériau matriciel comprend d'environ 0,1% à environ 15% de matériau matriciel par rapport au poids de la solution.

12. Méthode selon la revendication 11, dans laquelle ladite solution de matériau matriciel comprend d'environ 0,1% à environ 3% en poids dudit agent formant la matrice.

13. Méthode selon la revendication 11, dans laquelle ladite solution de matériau matriciel comprend d'environ 0,5% à environ 10% en poids desdits un ou plusieurs amino-acides et d'environ 0,5% à environ 10% en poids de mannitol.

14. Méthode selon l'une quelconque des revendications 10 à 13, dans laquelle la forme galénique sans ingrédient actif est d'abord préparée et ensuite chargée d'une quantité prédéterminée de l'ingrédient actif.

15. Méthode selon l'une quelconque des revendications 10 à 14, dans laquelle la solution de matériau matriciel comprend en outre un gaz qui y est dispersé en vue de former une forme galénique alvéolaire.

16. Forme galénique ou méthode selon l'une quelconque des revendications précédentes, dans laquelle ledit rapport pondéral entre l'agent formant la matrice et lesdits un ou plusieurs amino-acides est de 3:1 à 1:1.

17. Forme galénique ou méthode selon la revendication 16, dans laquelle ledit rapport pondéral est de 1,5:1 à 1:1.

18. Forme galénique ou méthode selon l'une quelconque des revendications 1 à 15, dans laquelle ledit rapport pondéral entre l'agent formant la matrice et lesdits un ou plusieurs amino-acides est de 1,5:1 à 1:5.
